(19) Europäisches Patentamt
European Patent Office
Office européen des brevets

(11) **EP 2 763 652 B1**

(12) **FASCICULE DE BREVET EUROPEEN**

(45) Date de publication et mention
de la délivrance du brevet:
**06.12.2017 Bulletin 2017/49**

(51) Int Cl.:
**A61K 8/73** (2006.01)   **A61K 8/97** (2017.01)
**A61Q 19/08** (2006.01)

(21) Numéro de dépôt: **12775812.6**

(22) Date de dépôt: **02.10.2012**

(86) Numéro de dépôt international:
**PCT/FR2012/052231**

(87) Numéro de publication internationale:
**WO 2013/050697 (11.04.2013 Gazette 2013/15)**

(54) **UTILISATION DE GLUCANES OBTENUS A PARTIR DE PRUNUS PERSICA COMME AGENT COSMETIQUE ANTI-AGE**

VERWENDUNG VON AUS PRUNUS PERSICA GEWONNENEN GLUCANEN ALS KOSMETISCHE ANTI-AGING-MITTEL

USE OF GLUCANS OBTAINED FROM PRUNUS PERSICA AS AN ANTI-AGING COSMETIC AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.10.2011 FR 1158912**

(43) Date de publication de la demande:
**13.08.2014 Bulletin 2014/33**

(73) Titulaire: **Societe Industrielle Limousine d'Application Biologique**
**19130 Objat (FR)**

(72) Inventeur: **PAUFIQUE, Jean**
**F-19130 Objat (FR)**

(74) Mandataire: **Aquinov**
**Allée de la Forestière**
**33750 Beychac et Caillau (FR)**

(56) Documents cités:
**WO-A2-2011/027085**

- Lucia Merlo ET AL: "Changes in carbohydrate and enzyme levels during development of leaves of Prunus persica, a sorbitol synthesizing species", Physiologia Plantarum, 1 avril 1991 (1991-04-01), pages 621-626, XP055028995, DOI: 10.1111/j.1399-3054.1991.tb02478.x Extrait de l'Internet: URL:http://onlinelibrary.wiley.com/store/10.1111/j.1399-3054.1991.tb02478.x/asset/j.1399-3054.1991.tb02478.x.pdf?v=1&t=h3327apx&s=78e35d826990f40f8ae7bd8db7779d10ef9860 27 [extrait le 2012-02-06]
- DATABASE WPI Week 200478 Thomson Scientific, London, GB; AN 2004-789965 XP002677416, & JP 2004 315481 A (EZAKI GLICO CO LTD) 11 novembre 2004 (2004-11-11)
- DATABASE WPI Week 200244 Thomson Scientific, London, GB; AN 2002-409389 XP002677417, & JP 2002 034587 A (DOKURITSU GYOSEI HOJIN NOGYO SEIBUTSU SH) 5 février 2002 (2002-02-05)
- CALDWELL D L ET AL: "Chemical analysis of peach extrafloral nectary exudate", PHYTOCHEMISTRY, PERGAMON PRESS, GB, vol. 25, no. 2, 22 janvier 1986 (1986-01-22), pages 411-413, XP026633738, ISSN: 0031-9422, DOI: 10.1016/S0031-9422(00)85491-6 [extrait le 1986-01-22]

EP 2 763 652 B1

- **JAN NADWODNIK ET AL: "Subcellular concentrations of sugar alcohols and sugars in relation to phloem translocation in Plantago major, Plantago maritima, Prunus persica, and Apium graveolens", PLANTA ; AN INTERNATIONAL JOURNAL OF PLANT BIOLOGY, SPRINGER, BERLIN, DE, vol. 227, no. 5, 9 janvier 2008 (2008-01-09), pages 1079-1089, XP019590200, ISSN: 1432-2048**
- **DATABASE WPI Week 201082 Thomson Scientific, London, GB; AN 2010-M41815 XP002677418, & KR 2010 0060940 A (UNIV KOREA IND&ACAD COOP FOUND) 7 juin 2010 (2010-06-07)**

Remarques:
Le dossier contient des informations techniques présentées postérieurement au dépôt de la demande et ne figurant pas dans le présent fascicule.

**Description**

La présente invention se rapporte à l'utilisation de molécules spécifiques obtenues à partir de feuilles de *Prunus persica,* ainsi qu'à un principe actif particulier contenant de telles molécules, pour une action cutanée antivieillissement par activation des vitagènes.

L'invention concerne également les compositions cosmétiques incluant de telles molécules, et un procédé de traitement cosmétique destiné à atténuer les signes du vieillissement cutané.

La peau possède un système d'adaptation au stress pour faire face aux diverses agressions internes ou externes qui l'affectent quotidiennement. Différents mécanismes cellulaires de réponse au stress sont continuellement activés pour induire une réponse adaptative temporaire qui permet aux cellules de maintenir leurs fonctions biologiques face à leur environnement et de garantir ainsi l'homéostasie cutanée.

Toutefois, l'épuisement progressif des mécanismes protecteurs d'adaptation par le stress quotidien, intense ou répété, entraine le déclin graduel des fonctions cellulaires et la rupture de l'homéostasie. L'inefficacité des cellules à répondre et à s'adapter à leur environnement se traduit alors par le vieillissement de la peau.

Pour lutter contre les signes du vieillissement de la peau, on recherche donc à améliorer la capacité de réponse au stress des cellules cutanées.

Pour ce faire, une des voies récemment étudiées est la voie hormétique. Le principe est basé sur la constatation selon laquelle un stress, s'il est modéré, peut également stimuler positivement l'organisme. En effet, en activant les mécanismes endogènes de défense et de réparation, un stress modéré arme les cellules à mieux combattre un stress ultérieur.

L'activation de cette réponse au stress nécessite l'induction d'un groupe de gènes protecteurs, les vitagènes. Ces derniers sont impliqués dans la préservation de l'homéostasie des cellules en cas d'agressions car ils codent pour des protéines de résistance au stress telles que les protéines chaperonnes type heat shock proteins (HSP), la thioredoxine et les sirtuines. On sait que le maintien de l'activité de ces vitagènes permet de limiter les désordres cellulaires intervenant avec l'âge.

C'est pourquoi, l'objectif de la présente invention est de proposer un principe actif cosmétique qui soit capable de mimer un micro-stress positif pour favoriser l'induction des vitagènes et armer les cellules de la peau à mieux combattre les agressions ultérieures.

Pour y répondre, l'invention propose d'utiliser des molécules particulières obtenues à partir de feuilles de *Prunus persica.*

Le pêcher *Prunus persica* est un arbre fruitier originaire d'Asie, cultivé dans le bassin méditerranéen depuis l'Antiquité et implanté en Europe à partir du Moyen-âge. Aujourd'hui le pêcher est largement cultivé sur tous les continents pour la récolte du fruit. Accessoirement, les feuilles de pêchers sont récoltées sur les plantations, comme c'est notamment le cas au Maroc.

En dehors de la consommation des fruits, le principal usage traditionnel marocain du pêcher concerne le noyau du fruit, et plus particulièrement l'amande, pour éclaircir le teint et adoucir la peau.

En Tunisie, les feuilles sont utilisées comme agent hypotenseur. Elles soignent également les otites, les abcès et elles ont des propriétés diurétiques.

A la Réunion, on utilise le coeur de pêches (feuilles qui entourent le bourgeon lors de la floraison de pêcher) en tisanes contre les douleurs abdominales des bébés. De manière générale, les feuilles sont peu utilisées par rapport aux fruits. On leur trouve parfois des vertus toniques, apéritives et digestives, diaphorétiques, purgatives, émollientes, diurétiques, expectorantes et fébrifuges. En outre, certains extraits ont été décrits pour des applications cosmétiques, notamment dans les demandes de brevet KR-20050026297, KR-20100010316 ou JP 2001187725, mais aucun ne répond à l'objectif de l'invention.

Par ailleurs, dans la demande WO2011/027085 il est décrit un principe actif issu d'une levure, Candida saitoana, comprenant des carbohydrates dont des a-glucanes, et son utilisation pour détoxifier les cellules de la peau en agissant sur l'autophagie des cellules cutanées.

La présente invention vise spécifiquement l'utilisation cosmétique d'a-glucanes de degré de polymérisation compris entre 2 et 13 obtenus à partir de feuilles de *Prunus persica* ou d'un principe actif en contenant, comme agent cosmétique dans une composition cosmétique, l'agent et/ou la composition destiné(s) à stimuler l'expression des vitagènes dans les cellules de la peau.

En effet, de façon surprenante l'utilisation d'a-glucanes de degré de polymérisation compris entre 2 et 13 obtenus à partir de feuilles de *Prunus persica* provoque un stress, qui, à dose modérée, en activant les mécanismes de défense et de réparation de la peau, permet d'entraîner une stimulation positive et bénéfique des cellules qui voient leurs performances globales améliorées.

Grâce à cet effet hormétique, le vieillissement de la peau est limité.

De façon spécifique, l'invention vise également un principe actif cosmétique particulier à savoir un hydrolysat de feuilles de *Prunus persica* comprenant des a-glucanes de degré de polymérisation compris entre 2 et 13.

Par « hydrolysat » on entend tout extrait obtenu à partir de feuilles de *Prunus persica,* comprenant au moins une étape d'hydrolyse enzymatique ou chimique, préférentiellement enzymatique.

Enfin, l'invention a également pour objet une composition cosmétique contenant des a-glucanes de degré de polymérisation compris entre 2 et 13 obtenus à partir de feuilles de *Prunus persica,* ainsi qu'un procédé de traitement cosmétique destiné à atténuer les signes de vieillissement de la peau comprenant l'application topique d'une telle composition.

La présente invention est maintenant décrite en détail.

UTILISATION

Selon un premier aspect, l'invention vise l'utilisation d'a-glucanes de degré de polymérisation compris entre 2 et 13 obtenus à partir de feuilles de *Prunus persica* ou d'un principe actif en contenant comme agent actif cosmétique dans une composition à application topique, préférentiellement une composition cosmétique, ledit agent actif et/ou ladite composition étant destiné(s) à favoriser l'induction des gènes des cellules de la peau type HSP72, HSP32, thiorédoxine, sirtuine-1 appelés vitagènes.

En effet, les a-glucanes de degré de polymérisation compris entre 2 et 13 obtenus à partir de feuilles de *Prunus persica* ou les principes actifs en contenant, lorsqu'ils sont appliqués sur la peau, miment un faible stress (stress modéré) qui active les voies hormétiques aboutissant à un programme de survie cellulaire programmé complexe : adaptation métabolique et production d'une panoplie de protéines anti-stress spécifiques qui permettent de résister aux conditions environnantes ultérieures plus drastiques. L'activation de ce programme résulte de diverses voies de signalisation régulées notamment par des facteurs de transcription centraux qui conduisent à l'induction des vitagènes. Ainsi, selon l'invention, l'utilisation d'a-glucanes de degré de polymérisation compris entre 2 et 13 obtenus à partir de feuilles de *Prunus persica* ou d'un principe actif en contenant sur les cellules cutanées, permet de stimuler l'expression de gènes, en particulier des gènes codant pour les protéines protectrices suivantes :

- les HSP, en particulier HSP72 et HSP32, qui sont des protéines chaperonnes moléculaires dont le rôle crucial est le maintien de l'homéostasie des protéines ; elles sont garantes de la bonne conformation des protéines dans des conditions basales et les protègent en cas de stress ; fortement inductibles, elles minimisent l'agrégation des protéines dénaturées en rétablissant leur bon repliement ou en les éliminant par la voie du protéasome ;
- la thioredoxine, qui est une protéine ubiquitaire majoritairement exprimée dans le cytoplasme et qui appartient au système de défense anti-oxydant jouant un rôle clé dans l'environnement rédox cellulaire ; la présence de thioredoxine protège les fibroblastes humains de la sénescence prématurée ;
- la sirtuine-1 (SIRT-1), qui est une protéine nucléaire régulant la déacétylation des histones et un régulateur clé de la survie cellulaire ; elle joue un rôle central dans de nombreuses voies cellulaires impliquées notamment dans le métabolisme cellulaire, le stress et la restriction calorique et régule aussi plusieurs facteurs de transcription des voies de réponse au stress.

En stimulant l'expression des vitagènes codant pour ces protéines, l'utilisation d'a-glucanes de degré de polymérisation compris entre 2 et 13 obtenus à partir de feuilles de *Prunus persica* ou d'un principe actif en contenant sur les cellules cutanées, permet d'amplifier la capacité de réponse au stress des fibroblastes humains.

En particulier les a-glucanes de degré de polymérisation compris entre 2 et 13 obtenus à partir de feuilles de *Prunus persica* ou d'un principe actif en contenant peuvent être utilisés comme agent actif dans des compositions à application topique, préférentiellement des compositions cosmétiques, pour booster les fonctions vitales des cellules cutanées.

Avec l'âge, les fonctions physiologiques de la peau s'altèrent. Affaiblis, les fibroblastes perdent leur vitalité et leur dynamisme : leur métabolisme décroit et leur potentiel de prolifération et de migration ralentit progressivement. Ainsi, ce sont les capacités globales de régénération de la peau qui déclinent. En parallèle, les capacités de réponse au stress s'épuisent également entrainant, par un cercle vicieux, l'accélération du vieillissement.

Avantageusement, les a-glucanes de degré de polymérisation compris entre 2 et 13 obtenus à partir de feuilles de *Prunus persica* ou d'un principe actif en contenant peuvent être utilisés comme agent actif dans des compositions à application topique, en particulier des compositions cosmétiques, pour stimuler les capacités de prolifération et de migration des cellules cutanées. En effet, l'utilisation a-glucanes de degré de polymérisation compris entre 2 et 13 obtenus à partir de feuilles de *Prunus persica* ou d'un principe actif en contenant sur la peau, permet de stimuler la croissance cellulaire (viabilité et prolifération) et/ou le potentiel migratoire des cellules de la peau.

L'effet a-glucanes de degré de polymérisation compris entre 2 et 13 obtenus à partir de feuilles de *Prunus persica* sur les cellules cutanées est hormétique : à faible dose, ils favorisent les capacités de régénération des cellules tandis qu'ils les inhibent à des doses élevées.

Visuellement, l'utilisation d'a-glucanes de degré de polymérisation compris entre 2 et 13 obtenus à partir de feuilles de *Prunus persica* ou d'un principe actif en contenant sur les cellules cutanées, permet de lisser le microrelief en réduisant la rugosité et le volume des rides. La peau est régénérée et revitalisée, et les signes du vieillissement sont atténués.

L'invention vise donc l'utilisation d'a-glucanes de degré de polymérisation compris entre 2 et 13 obtenus à partir de feuilles de *Prunus persica* ou d'un principe actif en contenant comme agent actif cosmétique dans une composition à application topique, préférentiellement à application cosmétique, ledit agent actif et/ou ladite composition étant destiné(s) à atténuer les signes du vieillissement cutané. Selon un mode de réalisation particulièrement adapté, l'invention vise l'utilisation d'un principe actif obtenu à partir de feuilles de *Prunus persica* contenant des a glucanes de degré de polymérisation compris entre 2 et 13, tel que décrit en suivant.

PRINCIPE ACTIF

L'invention concerne également un principe actif cosmétique particulier, à savoir un hydrolysat de feuilles de *Prunus persica* comprenant des a-glucanes de degré de polymérisation compris entre 2 et 13.

Les a-glucanes de degré de polymérisation compris entre 2 et 13 présentent une masse moléculaire inférieure à 2300 Da.

De façon préférée les des a-glucanes de degré de polymérisation compris entre 2 et 13 représentent au moins 9% en poids de matière sèche du principe actif. Le principe actif selon l'invention est préférentiellement un hydrolysat enzymatique.

Le principe actif présente préférentiellement une couleur jaune clair.

Il peut se présenter sous forme liquide et être défini par au moins une des caractéristiques exposées ci-après, préférentiellement toutes.

Matières sèches :

Le taux de matières sèches d'un principe actif selon l'invention (mesuré par passage à l'étuve à 105°C en présence de sable d'un échantillon de poids initial donné jusqu'à obtention d'un poids constant) peut être compris entre 10 et 120 g/l, préférentiellement entre 26 et 40 g/l.

Mesure du pH :

Le pH (mesuré par la méthode potentiométrique à température ambiante) peut être compris entre 3,6 et 6,5, préférentiellement entre 4,5 et 5,5.

Carbohydrates :

Détermination de la teneur en sucres totaux

Le dosage de la teneur en sucres totaux peut être réalisé par la méthode de DUBOIS (DUBOIS M. et al., (1956), Analytical chemistry, 28, n°3 p. 350-356). En présence d'acide sulfurique concentré et de phénol, les sucres réducteurs donnent un composé jaune orangé. A partir d'une gamme étalon, on peut déterminer le taux de sucres totaux d'un échantillon. La teneur en sucres totaux peut être comprise entre 1,5 et 24g/l, préférentiellement entre 4 et 8g/l.

Caractérisation des carbohydrates

Les masses moléculaires des carbohydrates présents dans le principe actif selon l'invention sont déterminées grâce à la chromatographie en phase liquide haute performance (CLHP).

La fraction glucidique du principe actif selon l'invention est majoritairement composée d'a-glucanes de degré de polymérisation compris entre 2 et 13. Les a-glucanes de degré de polymérisation compris entre 2 et 13 représente préférentiellement entre 9 et 15% en poids de matière de sèche du principe actif.

Protéines

Détermination de la teneur en protéines :

Le dosage de la teneur en protéines est réalisé selon la méthode de LOWRY (Lowry et al. Protein measurement with the folin reagent, J. Biol. Chem, 193, 265-275, 1951). Le réaction de Folin donne une coloration bleue avec certains acides aminés. La coloration obtenue est comparée avec celle d'une courbe établie avec un sérum étalon.

La teneur en protéines peut aussi être exprimée en pourcentage par rapport à la matière sèche. Elle représente donc préférentiellement moins de 10% en poids par rapport à la matière sèche.

Caractérisation de la fraction protéique :

La caractérisation de la fraction protéique du principe actif selon l'invention est réalisée par exclusion stérique F.P.L.C (Fast Protein Liquid Chromatography). L'étalonnage de la colonne est réalisé par le passage de marqueurs de masse moléculaire définie (Cytochrome C, Aprotinine, Vitamine B12 et Cytidine).
Les protéines du principe actif selon l'invention sont composées de peptides de masses molaires inférieures à 2000Da.

Teneur en cendres brutes :

La teneur en cendres brutes est déterminée par la pesée des résidus issus de l'incinération à 550°C dans un four à moufle électrique (VULCAN™).
Le poids du résidu est calculé en déduisant la tare.
La teneur en minéraux est exprimée en pourcentage par rapport au poids total de la matière sèche du principe actif.
La teneur en cendres brutes d'un principe actif selon l'invention est préférentiellement comprise entre 28 et 38%.

Teneur en acides uroniques :

La teneur en acides uroniques est déterminée selon la méthode suivante.
Le produit de l'acide galacturonique avec le tétraborate de sodium, donne en présence du méta-hydroxydiphényl, une coloration rose permettant le dosage au spectrophotomètre à 520nm.
L'intensité de coloration est proportionnelle à la quantité d'acides uroniques. Les lectures sont réalisées à partir d'une gamme étalon d'acide galacturonique allant de 10 à 100 mg/l.
La teneur en acides uroniques d'un principe actif selon l'invention est comprise entre 2 et 6% par rapport au poids total de la matière sèche du principe actif.

Teneur en composés phénoliqiues :

Les composés phénoliques forment en présence de ferricyanure de potassium des composés colorés, détectables à 715nm. L'intensité de coloration est proportionnelle à la quantité de composés phénoliques.
Les lectures sont réalisées à partir d'une gamme étalon d'acide gallique allant de 40 à 120 mg/l. Les résultats obtenus pour les étalons permettent de tracer une droite densité optique en fonction de la concentration et le taux de polyphénols des échantillons est lu directement sur cette droite.
La teneur en composés phénoliques d'un principe actif selon l'invention est inférieure à 1% en pourcentage par rapport à la matière sèche.

Identification de la fraction active

Afin de démontrer que la fraction majoritairement active du principe actif selon l'invention est bien la fraction constituée par les a-glucanes de degré de polymérisation compris entre 2 et 13, une étude a été réalisée. Cette étude consiste à fractionner les espèces moléculaires du principe actif selon l'invention :

-   une fraction A constituée de cendres, obtenue par ressolubilisation des résidus issus de l'incinération à 550°C dans un four à moufle électronique, reprise dans de l'eau distillée et filtrée,
-   une fraction B contenant des carbohydrates neutres purifiés par adsorption des composés cationiques et anioniques du principe actif. La fraction B contient 84% des sucres et 10% des protéines du principe actif selon l'invention,
-   une fraction C constituée de sucres libres obtenus par hydrolyse des carbohydrates en sucres simples, et des protéines du principe actif. L'étude consiste à comparer l'effet de ces différentes fractions sur l'expression de HSP72 par PCR quantitative sur fibroblastes humains normaux, au résultat obtenu pour le principe actif selon l'invention à 1%. Le protocole de l'essai est celui décrit au point I.a.

Les résultats obtenus sont présentés dans le tableau ci-après :

| | Taux de collagène I / Témoin (%) |
|---|---|
| Principe actif à 1% | +22% |
| Fraction A 1% | -3% |
| Fraction B 1% | +29% |
| Fraction C 1% | -10% |

Les fractions A et C ne sont pas efficaces. C'est la fraction B qui confère au principe actif son activité.

L'analyse de la fraction B par HPLC (Chromatographie Liquide Haute Performance), montre qu'elle est constituée majoritairement d'a-glucanes de degré de polymérisation compris entre 2 et 13 (poids moléculaire inférieur à 2300 Da).

Ce sont donc bien les a-glucanes de degré de polymérisation compris entre 2 et 13 qui confèrent au principe actif son efficacité.

PROCEDE D'OBTENTION

Le principe actif selon l'invention tel que décrit précédemment peut être obtenu préférentiellement par un procédé comprenant une hydrolyse enzymatique.

Un procédé particulièrement adapté comprend au moins la succession des étapes suivantes :

- solubilisation de poudre de feuilles de *Prunus persica* dans une solution aqueuse, préférentiellement à raison d'au moins 20g/l
- hydrolyse enzymatique, préférentiellement à l'aide d'une ou plusieurs carbohydrases,
- séparation des phases soluble et insoluble,
- inactivation enzymatique par traitement thermique de la phase soluble,
- filtration(s) successive(s) permettant de conserver les alpha-glucanes de degré de polymérisation inférieur ou égal à 13,
- filtration stérilisante.

Des étapes de clarification ou de décoloration ou de désodorisation peuvent être ajoutées.

Les paramètres des différentes étapes doivent être ajustés afin d'obtenir des principes actifs comprenant des a-glucanes de degré de polymérisation compris entre 2 et 13, en particulier un principe actif comprenant au moins 9% d'a-glucanes de degré de polymérisation compris entre 2 et 13.

COMPOSITIONS COSMETIQUES ET PROCEDE COSMETIQUE DE SOIN DE LA PEAU

La présente invention couvre aussi les compositions cosmétiques incluant des a-glucanes de degré de polymérisation compris entre 2 et 13 obtenus à partir de feuilles de *Prunus persica* ou un hydrolysat de feuilles de *Prunus persica* comprenant des a-glucanes de degré de polymérisation compris entre 2 et 13, dans différentes formes galéniques, adaptées à l'administration par voie topique cutanée.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect d'une crème, d'une lotion, d'un lait, d'un sérum, d'une pommade, d'un gel, d'une pâte ou d'une mousse, ou sous forme solide.

Il peut s'agir de compositions comprenant entre 0,01 et 3% de principe(s) actif(s) issu(s) de feuilles de *Prunus persica* selon la présente invention.

Ces compositions comprennent, outre l'actif, un milieu physiologiquement acceptable et de préférence cosmétiquement acceptable, c'est-à-dire qui ne provoque pas de sensations d'inconfort inacceptables pour l'utilisateur tels que des rougeurs, tiraillements ou picotements.

Les compositions selon l'invention peuvent contenir comme adjuvant au moins un composé choisi parmi :

- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba ;

- les élastomères de silicone,
- les tensioactifs, de préférence émulsionnants, qu'ils soient non ioniques, anioniques, cationiques ou amphotères ;
- les co-tensioactifs, tels que les alcools gras linéaires ;
- les épaississants et/ou gélifiants,
- les humectants, tels que les polyols comme la glycérine ;
- les filtres organiques,
- les filtres inorganiques,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA (International Cosmetic Ingrédient Dictionary and Handbook publié par le Personal Care Product Council).

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à atténuer les signes du vieillissement cutané.

L'invention vise à cet effet un procédé cosmétique de soin de la peau humaine, destiné à atténuer les signes du vieillissement cutané, comprenant l'application topique d'une composition renfermant des a-glucanes de degré de polymérisation compris entre 2 et 13 obtenus à partir de feuilles de *Prunus persica* ou un principe actif en contenant ou une composition comprenant entre 0,01 et 3% de principe(s) actif(s) issu(s) de feuilles de *Prunus persica* selon la présente invention. Le procédé cosmétique de soin de la peau selon l'invention peut être destiné à stimuler la croissance cellulaire et/ou le potentiel migratoire des cellules cutanées et/ou à favoriser la capacité de régénération de la peau et/ou à lisser le microrelief cutané et/ou à favoriser l'induction des gènes de HSP72, de HSP32, de thiorédoxine et/ou de sirtuine-1 des cellules de la peau.

EXEMPLES

Un exemple non limitatif de procédé d'obtention, de principe actif contenant des a-glucanes obtenu à partir de feuilles de *Prunus persica*, est présenté en suivant, ainsi que des exemples de composition incluant un tel principe actif.

Exemple 1 : procédé d'obtention du principe actif selon l'invention

Un exemple de procédé d'obtention d'un principe actif selon l'invention, comprend la mise en oeuvre des étapes suivantes :

- solubilisation de poudre de feuilles de *Prunus persica* dans une solution aqueuse à raison de 20g/l,
- hydrolyse enzymatique des carbohydrates,
- séparation des phases soluble et insoluble
- récupération de la phase soluble et traitement thermique pour inactiver les activités enzymatiques résiduelles,
- deux filtrations successives permettant de conserver les alpha-glucanes de degré de polymérisation inférieur ou égal à 13.
- ajout d'un adjuvant pour favoriser la décoloration,
- filtration et filtration stérilisante.

Le principe actif obtenu présente les caractéristiques suivantes :

- aspect : liquide limpide
- couleur : jaune clair
- teneur en matières sèches : 32,1g/l
- pH : 5,1
- teneur en sucres totaux : 55,7 % en poids par rapport à la matière sèche,
- teneur en cendres : 32,6% en poids par rapport à la matière sèche
- teneur en protéines totaux : 8,6% en poids par rapport à la matière sèche,
- teneur en acides uroniques : 4,0% en poids par rapport à la matière sèche
- teneur en polyphénols : 0,1% en poids par rapport à la matière sèche.

Exemple 2 : utilisation d'un principe actif selon l'invention dans une crème légère

| Phase A. | Eau | QSP 100% |
|---|---|---|
| | Glycérol | 2,6% |
| | Butylène glycol | 6% |
| Phase B. | Cethyl Alcool | 1% |
| | Rita GMS (Rita) | 1% |
| | Stéaryl Alcool | 1% |
| | DUB MDIS (Stéarinerie DUBOIS) | 5% |
| | DUB MCT 5545 (Stéarinerie DUBOIS) | 5% |
| Phase C. | Principe actif selon l'invention (exemple 1) | 3% |
| | Satiaxane CX930 (Cargill) | 0,17% |
| | Titane dioxide | 0,67% |
| | Conservateur | 0,7% |

Les quantités indiquées sont données en pourcentage de poids.
Cette émulsion fluide blanche présente un pH de 5,6. En application topique, elle présente une pénétration rapide avec un fini doux et sec.
Elle peut être obtenue par la mise en oeuvre des étapes suivantes :

- mélanger A, chauffer au bain marie à 80°C sous agitation magnétique,
- mélanger B, chauffer au bain marie à 80°C sous agitation magnétique,
- émulsionner B dans A sous rotor stator à 1200 tours/minute
- à 40°C, ajouter C, dans l'ordre indiqué sous rotor stator à 2400 tours/minute, et laisse refroidir jusqu'à complète homogénéisation des poudres.

Exemple 3 : utilisation d'un principe actif selon l'invention dans une crème soin

| Phase A. | Eau | QSP 100 % |
|---|---|---|
| | Chlorohydrol 50% (Reheis) | 10 % |
| Phase B. | DUB SEG (Stéarinerie DUBOIS) | 3% |
| | Biophylic H (Luca Meyer) | 1% |
| | Simulsol CS (Seppic) | 1% |
| | DUB MDIS (Stéarinerie DUBOIS) | 6% |
| | Montanov S (Seppic) | 3% |
| | Palmytate Cethyl | 1% |
| | DUB PPH1 (Stéarinerie DUBOIS) | 4% |
| | DUB APRILOSE (Stéarinerie DUBOIS) | 3% |
| Phase C. | Conservateur | 0,7 % |
| | Principe actif selon l'invention (exemple 1) | 3% |
| | Talc (Luzenac) | 0,67% |

Les quantités indiquées sont données en pourcentage de poids.
Cette émulsion blanche onctueuse présente un pH de 5. En application topique, elle présente un effet doux avec un fini sec légèrement poudré.
Elle peut être obtenue par la mise en oeuvre des étapes suivantes :

- mélanger A, mélanger B,
- chauffer A et B séparément à 80°C sous agitation magnétique,
- émulsionner B dans A sous émulseur rotor stator à 1500 tours/minute,

- à 40°C, additionner C dans l'ordre,
- laisser sous agitation jusqu'à complète homogénéisation.

Exemple 4 : utilisation d'un principe actif selon l'invention dans une émulsion pour renouvellement cellulaire

| Phase A. | Eau | QSP 100 % |
| | Propylène glycol | 4% |
| Phase B. | Lanol 99 (Seppic) | 7% |
| | Montanov 202 (Seppic) | 5% |
| | Lanol 1688 (Seppic) | 4% |
| | Simulsol 165 (Seppic) | 2% |
| | Cethyl alcohol (Stéarinerie Dubois) | 1% |
| Phase C. | Conservateur | 0,7% |
| | Principe actif selon l'invention (exemple 1) | 3% |

Les quantités indiquées sont données en pourcentage de poids.
Cette émulsion à application topique présente un pH de 6,6.
Elle peut être obtenue par la mise en oeuvre des étapes suivantes :

- mélanger A, chauffer au bain-marie à 80°C sous agitation magnétique,
- mélanger B, chauffer au bain-marie à 80°C sous agitation magnétique,
- émulsionner B dans A sous émulseur rotor stator à 1500 tours/minute,
- à 40°C, ajouter C dans l'ordre indiqué sous rotor stator à 2400 tours/minute,
- laisser refroidir jusqu'à complète homogénéisation.

Exemple 5 : utilisation d'un principe actif selon l'invention dans une crème anti-rides

| Phase A. | Eau | QSP 100 % |
| Phase B. | Lanol 99 (Seppic) | 5% |
| | Montanov 202 (Seppic) | 3% |
| | Montanov 68 (Seppic) | 2% |
| Phase C. | Conservateur | 0,7% |
| | Principe actif selon l'invention (exemple 1) | 3% |
| Phase D. | Sepigel 305 (Seppic) | 0,3% |

Les quantités indiquées sont données en pourcentage de poids.
Cette crème à application topique présente un pH de 6,5.
Elle peut être obtenue par la mise en oeuvre des étapes suivantes :

- mélanger A, chauffer au bain-marie à 80°C sous agitation magnétique,
- mélanger B, chauffer au bain-marie à 80°C sous agitation magnétique,
- émulsionner B dans A sous émulseur rotor stator à 1500 tours/minute,
- à 40°C, ajouter C dans l'ordre indiqué sous rotor stator à 1500 tours/minute,
- à 30°C, ajouter D sous pâle mécanique à 2000 tours/minute,
- laisser agiter jusqu'à complet refroidissement.

EVALUATION DE L'EFFICACITE COSMETIQUE D'UN PRINCIPE ACTIF SELON L'INVENTION

**A. Tests in vitro**

I. Etude de l'effet sur l'expression des vitagènes

a-Evaluation de l'expression des ARNm codant pour HSP72, HSP32, thiorédoxine et sirtuine-1

Le but de l'étude est d'évaluer la capacité d'un principe actif selon l'invention (exemple 1) à augmenter l'expression des vitagènes codant pour :

- Les HSP72 et HSP32, protéines chaperonnes impliquées dans la réponse au stress,
- La thiorédoxine : protéine anti-oxydante,
- La sirtuine-1 (SIRT-1) : protéine impliquée dans la longévité cellulaire.

L'étude a été réalisée sur fibroblastes humains normaux par PCR quantitative, selon le protocole opératoire décrit en suivant.

A J1 les fibroblastes humains normaux obtenus à partir de plusieurs donneurs sont ensemencés dans un milieu MEM complet. Les cellules sont ensuite incubées dans une étuve à 37°C.

A J3, le milieu de culture est éliminé et remplacé par du milieu contenant le principe actif selon l'invention à 0,5%, 1% ou 2% (V/V). Les cellules sont ensuite incubées à 37°C.

A J4, les cellules sont récupérées et les ARN totaux extraits.

Les ARN extraits ont été reverse-transcripts et les ADN complémentaires obtenus analysés par la technique de PCR quantitative. Les ARNm (ARN messager) de HSP72, HSP32, thiorédoxine et SIRT-1 ont été analysés.

Les résultats obtenus en pourcentage d'ARNm de HSP72, HSP32, thiorédoxine et SIRT-1 sont présentés dans le tableau ci-dessous :

| | Taux d'ARNm (%) | | | |
|---|---|---|---|---|
| | HSP72 | HSP32 | Thiorédoxine | SIRT-1 |
| Témoin | 100 | 100 | 100 | 100 |
| Principe actif selon l'invention 0,5% | 114 | 109 | 107 | 113 |
| Principe actif selon l'invention 1% | 122 | 131 | 117 | 130 |
| Principe actif selon l'invention 2% | 130 | 152 | 130 | 133 |

Ces résultats montrent sur fibroblastes humains normaux, que le principe actif selon l'invention augmente l'expression des gènes codant pour :

- les protéines chaperonnes de choc thermique HSP72 et HSP32,
- la thiorédoxine, protéine anti-oxydante, et
- la sirtuine-1 (SIRT-1), protéine impliquée dans la longévité cellulaire.

En particulier, testé à 1% sur fibroblastes humains normaux, le principe actif selon l'invention permet d'augmenter l'expression des gènes codant pour HSP72 de 22%, pour HSP32 de 31%, pour la thiorédoxine de 17% et pour SIRT-1 de 30%.

En stimulant l'expression des vitagènes, le principe actif selon l'invention, permet d'amplifier la capacité de réponse au stress des fibroblastes humains.

b-Evaluation de la synthèse de HSP72 sur explants de peau

Cette étude a pour but d'évaluer l'effet d'un principe actif selon l'invention sur la synthèse de HSP72, protéine chaperonne impliquée dans la réponse au stress. L'étude est réalisée par immunohistofluorescence sur explants de peau humaine, selon le protocole opératoire décrit en suivant.

Des punchs de 8mm de diamètre sont réalisés à partir de plastie humaine et maintenus en survie dans du milieu de culture.

Ces explants sont pré-traités topiquement avec le principe actif de l'exemple 1 formulé à 0,5%, 1% et 3% ou avec un placebo.

Les explants sont récupérés après 24 heures et congelés pour réaliser des coupes à l'aide d'un cryostat.

On réalise ensuite un marquage immunohistologique des HSP72, et on visualise les résultats à l'aide d'un microscope couplé à un système d'analyse d'images. L'intensité de la fluorescence (couleur verte) est proportionnelle à la synthèse des HSP72.

Les résultats immunohistologiques étant qualitatifs, 4 niveaux d'expression ont été définis :

- absence de détection d'immunoréactivité (couleur verte nulle) -
- faible détection d'immunoréactivité (couleur verte faible) +
- moyenne détection d'immunoréactivité (couleur verte moyenne) ++
- forte détection d'immunoréactivité (couleur verte intense) +++ Les résultats obtenus sont résumés dans le tableau ci-dessous :

|  | Synthèse des HSP72 |
|---|---|
| Placebo | + |
| Principe actif selon l'invention 0,5% | ++ |
| Principe actif selon l'invention 1% | +++ |
| Principe actif selon l'invention 3% | +++ |

Les résultats de cette étude montrent que sur explants de peau, le principe actif selon l'invention augmente la synthèse de HSP72, protéine chaperonne impliquée dans la réponse au stress.

II. Effet sur la croissance cellulaire

a-Etude de la viabilité

Cet essai a pour objectif d'évaluer l'effet hormétique d'un principe actif selon l'invention sur la croissance de fibroblastes humains normaux en milieu carencé en nutriments.

L'étude a été réalisée par la mesure de la viabilité cellulaire à l'aide d'une coloration au M.T.T. (bromure de 3-(4,5-dimethylthiazol-2-yl)-2,5-diphenyl tetrazolium) selon le protocole opératoire décrit ci-après.

A J1 des fibroblastes humains sont ensemencés dans un milieu de culture MEM complet et incubées à 37°C dans une étuve contenant 5% de $CO_2$.

A J2 le milieu de culture est éliminé et remplacé par un milieu de culture contenant de 0,1 à 30% (v/v) de principe actif de l'exemple 1 ou un témoin positif (SVF sérum de veau foetal 10%). Les cellules sont ensuite incubées pendant 72 heures à 37°C.

A J5 les cellules sont récupérées et une étude de la croissance cellulaire est réalisée par mesure de coloration M.T.T. (Densité Optique à 540nm).

Le pourcentage de viabilité cellulaire est déterminé comme suit :

$$\text{Viabilité cellulaire (\%)} = (\text{DO essai} / \text{DO témoin}) \times 100$$

Les résultats obtenus sont présentés dans le tableau suivant :

|  | Viabilité cellulaire (%) |
|---|---|
| Témoin | 100 |
| SVF 10% | 196 |
| Principe actif selon l'invention 0,1% | 102 |
| Principe actif selon l'invention 0,5% | 117 |
| Principe actif selon l'invention 1% | 122 |
| Principe actif selon l'invention 2,5% | 128 |

(suite)

|  | Viabilité cellulaire (%) |
|---|---|
| Principe actif selon l'invention 5% | 142 |
| Principe actif selon l'invention 10% | 118 |
| Principe actif selon l'invention 20% | 109 |
| Principe actif selon l'invention 30% | 97 |

Ces résultats montrent que le principe actif selon l'invention pour une dose inférieure à 20% permet d'augmenter la viabilité des fibroblastes humains normaux en milieu carencé.

On constate également que cet effet est dose-dépendant et hormétique.

b-Etude de capacité de prolifération

L'objectif de cette étude est d'évaluer l'effet hormétique d'un principe actif selon l'invention sur la capacité proliférative de fibroblastes humains normaux en milieu carencé en nutriments en étudiant la synthèse de Ki67.

L'étude a été réalisée par immunocytologie sur fibroblastes humains normaux, selon le protocole opératoire décrit en suivant.

A J1, les fibroblastes humains sont ensemencés sur des lames de verre dans du milieu de culture complet, puis incubées à 37°C.

Le milieu de culture est ensuite éliminé et remplacé par du milieu de culture contenant le principe actif de l'exemple 1 de 0,1 à 10% (v/v) ou un témoin positif (SVF 10%).

Les cellules sont ensuite incubées pendant 72 heures à 37°C dans une atmosphère humide contenant 5% de $CO_2$.

A J5, on réalise un marquage immunocytologique de Ki67, et on visualise les résultats sur un microscope couplé à un système d'analyse d'images. L'intensité du marquage du Ki67 est proportionnelle à l'intensité de fluorescence verte présente dans les noyaux cellulaires. Plus la fluorescence est importante, plus le taux de synthèse de Ki67 est élevé. Les noyaux cellulaires sont contre-colorés en bleu. La quantification est réalisée en dénombrant les cellules Ki67 positives (noyau vert) sur le nombre de cellules totales.

Les résultats obtenus sont présentés dans le tableau suivant :

|  | Taux de cellules Ki67 positives (%) | Capacité de prolifération (%) |
|---|---|---|
| Témoin | 35 | 100 |
| SVF 10% | 56 | 158 |
| Principe actif selon l'invention 0,1% | 43 | 121 |
| Principe actif selon l'invention 0,25% | 45 | 128 |
| Principe actif selon l'invention 0,5% | 47 | 132 |
| Principe actif selon l'invention 1% | 49 | 139 |
| Principe actif selon l'invention 2,5% | 45 | 127 |
| Principe actif selon l'invention 5% | 35 | 98 |
| Principe actif selon l'invention 10% | 16 | 45 |

Ces résultats montrent que le principe actif selon l'invention sur fibroblastes humains normaux, permet de stimuler la capacité de prolifération des fibroblastes humains de façon dose-dépendante et hormétique.

III. Effet sur le potentiel migratoire des cellules

Cette étude a pour objectif d'évaluer l'effet hormétique d'un principe actif selon l'invention sur la capacité de migration de fibroblastes humains normaux.

La migration des cellules a été évaluée par une observation microscopique après coloration sur des cultures de cellules en monocouche « blessées » par une rayure réalisée à l'aide d'une pipette. Le protocole opératoire de l'étude est le suivant.

A J1 les fibroblastes humains normaux sont ensemencés dans du milieu complet, puis incubés à 37°C.

A J4, le milieu de culture est éliminé et remplacé par du milieu dépourvu de SVF. A J5, on réalise une blessure des cellules à l'aide d'un pipette plastique 1ml. Le milieu de culture est éliminé et remplacé par un milieu contenant ou non le principe actif de l'exemple 1 de 0,1 à 20% (v/v). Les cellules sont incubées pendant 72 heures à 37°C.

A J8, le milieu de culture est éliminé. Le tapis cellulaire de fibroblastes est coloré à l'aide d'une solution de cristal violet à 0,5% (M/V).

La visualisation de la coloration est réalisée à l'aide d'un microscope couplé à un système d'analyse d'images. Les cellules apparaissent colorées spécifiquement en violet. Plus le marquage violet est présent dans la zone de blessure, plus la migration des cellules est importante.

Les résultats obtenus sont donnés dans le tableau ci-dessous :

|  | Migration cellulaire (%) |
|---|---|
| Témoin | 100 |
| Principe actif selon l'invention 0,1% | 102 |
| Principe actif selon l'invention 0,5% | 157 |
| Principe actif selon l'invention 1% | 183 |
| Principe actif selon l'invention 2,5% | 143 |
| Principe actif selon l'invention 5% | 104 |
| Principe actif selon l'invention 10% | 82 |
| Principe actif selon l'invention 20% | 74 |

Ces résultats montrent que testé à 1% le principe actif selon l'invention stimule la capacité de migration des fibroblastes humains normaux de 83%.

Un principe actif selon l'invention permet donc de booster le potentiel de régénération des cellules de façon dose-dépendante et hormétique.

III. Evaluation de l'activité anti-radicalaire

Le but de cette étude est démontrer que l'effet du principe actif selon l'invention n'est pas lié à une activité anti-radicalaire, en utilisant la méthode au DPPH.

Le DPPH (Diphényl-picrylhydrazylhydrate) est un radical libre, absorbant dans le violet à 517nm. Un produit anti-radicalaire entraîne une disparition de la coloration violette. L'activité anti-radicalaire d'une substance est exprimée en pourcentage d'inhibition de l'absorbance du DPPH.

Le protocole opératoire est le suivant.

A 200 $\mu$l du principe actif dilué au 1/20$^{ème}$, 1/50$^{ème}$ ou 1/100$^{eme}$, on ajoute 2500$\mu$l de la solution DPPH. Les essais sont maintenus pendant 15 minutes à l'obscurité. Les résultats obtenus sont présentés dans le tableau ci-dessous :

|  | Activité DPPH (%) |
|---|---|
| Principe actif selon l'invention 1% | 0,1% |
| Principe actif selon l'invention 2% | 0,1% |

Ces résultats montrent que le principe actif selon l'invention ne présente pas d'activité antiradicalaire significative selon la méthode du DPPH.

**B. Tests in vivo**

I. Effet régénérant sur la peau

Cette étude a pour objectif d'évaluer *in vivo* l'effet régénérant d'un principe actif selon l'invention formulé à 3% en émulsion contre placebo, grâce à l'étude du renouvellement cellulaire.

L'étude a été réalisée sur 18 volontaires sains, de sexe féminin, d'âge compris entre 28 et 50 ans.

Les mesures de la couleur de la peau ont été réalisées après coloration à la DHA (Dihydroxyacétone), à l'aide d'un Chromamètre® au niveau du ventre.

La coloration à la DHA est réalisé en appliquant sur chaque zone de mesure par massage léger 20 $\mu$l d'un gel contenant 8% de DHA. La mesure de la couleur obtenue est réalisée 72h après application du gel.

Les zones de mesure sont les suivantes :

- zone non traitée : application du gel de DHA sans aucun traitement par la suite
- zone placebo : application du gel de DHA suivie d'applications biquotidiennes de la formule placebo
- zone principe actif : application du gel de DHA suivie d'applications biquotidiennes de la formule de l'exemple 4 contenant 3% du principe actif de l'exemple 1.

Les mesures colorimétriques de la peau sont réalisées tous les 2 jours pendant 16 jours sur les différentes zones à l'aide d'un Chromamètre® qui convertit les couleurs situées dans la plage de perception humaine en un code numérique composé de trois paramètres :

- L* : paramètre de luminance qui représente la clarté (du sombre au pâle)
- a* : paramètre de chrominance qui représente la gamme des verts aux rouges
- b* : paramètre de chrominance qui représente la gamme des bleus aux jaunes.

Pour l'étude, seul le paramètre b*, caractéristique de la pigmentation jaune mélanique cutanée, est retenu : plus le paramètre b* diminue, plus la peau s'éclaircit, plus elle est régénérée.

Le protocole opératoire mis en oeuvre pour l'étude est le suivant.

Entre J-17 et J-3, aucune crème ne doit être appliquée au niveau du ventre.

A J-3, on détermine trois zones cutanées au niveau du ventre des volontaires, on mesure la couleur de la peau au Chromamètre® sur chaque zone, et on applique 20 $\mu$l de gel contenant 8% de DHA sur chacune des trois zones.

A J0, on mesure la couleur de la peau au Chromamètre® sur chacune des trois zones.

Entre J0 et J16, applications biquotidiennes du principe actif ou du placebo.

A J2, J4, J7, J9, J14 et J16, mesure de la couleur de la peau au Chromamètre® sur chaque zone.

Les résultats globaux obtenus sont présentés dans le tableau ci-dessous :

|  | J2-J0 | J4-J0 | J7-J0 | J9-J0 | J14-J0 | J16-J0 |
|---|---|---|---|---|---|---|
| Zone non traitée | -0,25 | -0,60 | -2,60 | -2,95 | -4,01 | -4,31 |
| Placebo | -0,09 | -0,91 | -2,96 | -3,23 | -4,03 | -4,19 |
| Principe actif selon l'invention 3% | -0,08 | -1,29 | -3,28 | -3,65 | -4,28 | -4,42 |

Dans les conditions de l'étude, on constate qu'après 16 jours d'applications biquotidiennes, en comparaison au placebo et après une coloration avec un gel de DHA, le principe actif selon l'invention formulé à 3% augmente la capacité de régénération de la peau en diminuant plus rapidement le paramètre b* représentatif de la couleur jaune mélanique.

II. Effet anti-rides sur la peau

Cette étude a pour objectif d'évaluer *in vivo* l'effet anti-rides d'un principe actif selon l'invention formulé à 3% en émulsion contre placebo, au niveau de la patte d'oie.

L'étude a été réalisée sur 18 volontaires sains, de sexe féminin, d'âge compris entre 46 et 65 ans, et présentant des rides au niveau des pattes d'oie.

Des acquisitions en 3 dimensions (3D) par projection de franges de la patte d'oie ont été réalisées. Les paramètres les plus pertinents retenus pour cette étude sont :

- des paramètres de rugosité en 3D

  • Sq : moyenne quadratique de rugosité de surface
  • Sa : moyenne arithmétique de rugosité de surface

- un paramètre de volume

- volume négatif : volume inférieur à la surface de la peau

Une diminution de ces différents paramètres est caractéristique d'une amélioration du relief de la surface étudiée et d'une diminution des rides.

Le protocole de l'étude est le suivant.

Entre J-14 et J0, les volontaires appliquent deux fois par jour sur l'ensemble du visage une crème placebo.

A J0, on réalise une acquisition en 3D de la patte d'oie par projection de franges. Entre J0 et J27, les volontaires appliquent biquotidiennement de principe actif selon l'invention et le placebo.

A J28, on réalise à nouveau des acquisitions 3D de la patte d'oie par projection de franges.

Les résultats globaux obtenus avec le principe actif selon l'invention en pourcentage de variation par rapport aux résultats obtenus avec le placebo sont présentés dans le tableau ci-dessous :

|  | Variation/placebo |
|---|---|
| Paramètre Sq | -3,9% |
| Paramètre Sa | -4,5% |
| Paramètre Volume négatif | -10,6% |

Dans les conditions de cette étude, après 28 jours d'applications biquotidiennes et en comparaison au placebo, le principe actif selon l'invention formulé à 3% en émulsion permet de lisser le relief cutané au niveau des pattes d'oie et de réduire les rides. En effet, le principe actif selon l'invention diminue les paramètres de rugosité (le paramètre Sq de 3,9% et le paramètre Sa de 4,5%) et le volume (-10,6%).

**Revendications**

1. Principe actif cosmétique obtenu à partir de feuilles de *Prunus persica,* **caractérisé en ce qu'**il s'agit d'un hydrolysat enzymatique de feuilles de *Prunus persica* comprenant au moins 9% d'a-glucanes de degré de polymérisation compris entre 2 et 13, en poids de matière sèche du principe actif.

2. Principe actif cosmétique selon la revendication 1, caractérisé en qu'il comprend entre 9% et 15% d'a-glucanes de degré de polymérisation compris entre 2 et 13, en poids de matière sèche du principe actif.

3. Principe actif cosmétique selon la revendication 1 ou 2, **caractérisé en ce qu'**il se présente sous forme liquide et **en ce qu'**il présente au moins une des caractéristiques suivantes :

    - un taux de matières sèches compris entre 26 et 40g/l,
    - une teneur en carbohydrates comprise entre 4 et 8g/l.

4. Composition cosmétique pour application topique, **caractérisée en ce qu'**elle comprend un principe actif selon l'une des revendications 1 à 3 présent entre 0,01 et 3% en poids total de la composition.

5. Procédé cosmétique de soin de la peau humaine, destiné à atténuer les signes du vieillissement cutané comprenant l'application topique d'une composition comprenant un principe actif selon l'une des revendications 1 à 3.

6. Procédé cosmétique de soin de la peau humaine pour favoriser la capacité de régénération de la peau et/ou à lisser le microrelief cutané comprenant l'application topique d'une composition comprenant un principe actif selon l'une des revendications 1 à 3.

7. Procédé cosmétique de soin de la peau humaine selon l'une des revendications 5 ou 6, **caractérisé en ce que** le principe actif permet de favoriser l'induction des gènes de HSP72, de HSP32, de thiorédoxine et/ou de sirtuine-1 des cellules de la peau.

8. Procédé cosmétique de soin de la peau humaine selon l'une des revendications 5 ou 6, **caractérisé en ce que** le principe actif permet de stimuler la croissance cellulaire et/ou le potentiel migratoire des cellules cutanées.

9.  Procédé cosmétique de soin de la peau humaine selon l'une des revendications 5 à 8, **caractérisé en ce que** la composition est la composition selon la revendication 4.

**Patentansprüche**

1.  Kosmetischer Wirkstoff, der aus Blättern von *Prunus persica* gewonnen ist, **dadurch gekennzeichnet, dass** es sich dabei um ein enzymatisches Hydrolysat der Blätter von *Prunus persica* mit in der Trockenmasse des Wirkstoffs wenigstens 9 Gew.-% von α-Glucanen mit einem Polarisationsgrad zwischen 2 und 13 handelt.

2.  Kosmetischer Wirkstoff nach Anspruch 1, **dadurch gekennzeichnet, dass** dieser in der Trockenmasse des Wirkstoffs zwischen 9 Gew.-% und 15 Gew.-% an α-Glucanen mit einem Polarisationsgrad zwischen 2 und 13 aufweist.

3.  Kosmetischer Wirkstoff nach Anspruch 1 oder 2, **dadurch gekennzeichnet, dass** dieser in flüssiger Form vorliegt und dass dieser wenigstens eines der folgenden Kennzeichen aufweist:

    - eine Trockenmasse zwischen 26 und 40 g/l,
    - einen Kohlenhydratgehalt zwischen 4 und 8 g/l.

4.  Kosmetischer Zusammensetzung für die topische Anwendung, **dadurch gekennzeichnet, dass** diese einen Wirkstoff nach einem der Ansprüche 1 bis 3 aufweist, der zwischen 0,01 und 3 % des Gesamtgewichts der Zusammensetzung ausmacht.

5.  Kosmetisches Verfahren zur Pflege der menschlichen Haut, das dazu bestimmt ist, die Anzeichen der Hautalterung abzuschwächen, und das die topische Anwendung einer Zusammensetzung mit einem Wirkstoff nach einem der Ansprüche 1 bis 3 umfasst.

6.  Kosmetisches Verfahren zur Pflege der menschlichen Haut, um die Fähigkeit zur Regeneration der Haut zu unterstützen und/oder das Mikrorelief der Haut zu glätten, das die topische Anwendung einer Zusammensetzung mit einem Wirkstoff nach einem der Ansprüche 1 bis 3 umfasst.

7.  Kosmetisches Verfahren zur Pflege der menschlichen Haut nach einem der Ansprüche 5 oder 6, **dadurch gekennzeichnet, dass** der Wirkstoff die Induktion der Gene HSP72 und HSP32, von Thioredoxin und/oder von Sirtuin-1 von Hautzellen unterstützt.

8.  Kosmetisches Verfahren zur Pflege der menschlichen Haut nach einem der Ansprüche 5 oder 6, **dadurch gekennzeichnet, dass** der Wirkstoff ermöglicht, das Zellwachstum und/oder das Migrationspotential der Hautzellen zu stimulieren.

9.  Kosmetisches Verfahren zur Pflege der menschlichen Haut nach einem der Ansprüche 5 bis 8, **dadurch gekennzeichnet, dass** die Zusammensetzung die Zusammensetzung nach Anspruch 4 ist.

**Claims**

1.  A cosmetic active principle obtained from leaves of *Prunus persica,* **characterised in that** it is an enzymatic hydrolysate of leaves of *Prunus persica* comprising at least 9% α-glucans with a degree of polymerisation of between 2 and 13, by weight of dry matter of the active principle.

2.  A cosmetic active principle according to claim 1, **characterised in that** it comprises between 9% and 15% α-glucans with a degree of polymerisation of between 2 and 13, by weight of dry matter of the active principle.

3.  A cosmetic active principle according to claim 1 or 2, **characterised in that** it is in liquid form and **in that** it has at least one of the following characteristics:

    - a degree of dry matter of between 26 and 40 g/l,
    - a carbohydrate content of between 4 and 8 g/l.

**4.** A cosmetic composition for topical application, **characterised in that** it comprises an active principle according to any of claims 1 to 3 present at between 0.01% and 3% by total weight of the composition.

**5.** A cosmetic method for care of the human skin, intended to attenuate signs of skin aging, comprising the topical application of a composition comprising an active principle according to any of claims 1 to 3.

**6.** A cosmetic method for care of the human skin for promoting the regeneration capability of the skin and/or smoothing cutaneous microrelief, comprising the topical application of a composition comprising an active principle according to any of claims 1 to 3.

**7.** A cosmetic method for care of the human skin according to either of claims 5 or 6, **characterised in that** the active principle makes it possible to promote the induction of the HSP72, HSP32, thioredoxin and/or sirtuin-1 genes of the skin cells.

**8.** A cosmetic method for care of the human skin according to either of claims 5 or 6, **characterised in that** the active principle makes it possible to stimulate cell growth and/or the migratory potential of skin cells.

**9.** A cosmetic method for care of the human skin according to any of claims 5 to 8, **characterised in that** the composition is the composition according to claim 4.

**EP 2 763 652 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- KR 20050026297
- KR 20100010316
- JP 2001187725 B
- WO 2011027085 A

### Littérature non-brevet citée dans la description

- **DUBOIS M. et al.** *Analytical chemistry,* 1956, vol. 28 (3), 350-356
- **LOWRY et al.** Protein measurement with the folin reagent. *J. Biol. Chem,* 1951, vol. 193, 265-275